# DEMANDE DE BREVET EUROPEEN

(11) **EP 1 892 015 A2**
(43) Date de publication de la demande: **27.02.2008**
(21) Numéro de dépôt: 07110932.6
(22) Date de dépôt: 25.06.2007
(51) Int. Cl.: A61Q 19/00, A61Q 19/02, A61Q 17/04, A61Q 19/08, A61K 8/81, A61K 8/73, A61K 8/894

(54) **Film à dissolution améliorée et produit cosmétique le contenant**

(30) Priorité: 10.08.2006 FR 0653343
(71) Demandeur: L'Oréal, 75008 Paris (FR)
(72) Inventeur: Cassin, Guillaume, 91140, VILLEBON SUR YVETTE (FR)
(74) Mandataire: Rasson, Catherine

(57) **Abrégé**

La présente invention se rapporte à un film anhydre hydrosoluble comportant (i) au moins un polymère filmogène hydrosoluble ou hydrodispersible, (ii) au moins un agent épaississant polysaccharidique, (iii) au moins un polysiloxane oxyalkyléné hydrosoluble ou hydrodispersible, et (iv) un ou plusieurs plastifiants choisis parmi les polyols.

L'invention se rapporte aussi au produit obtenu par mélange d'au moins un film avec une composition aqueuse, et à un kit de formulation d'un produit cosmétique comprenant une composition aqueuse et au moins un film anhydre, la composition aqueuse et le ou les films étant mélangés extemporanément en vue de former ledit produit cosmétique.

## Description

La présente invention se rapporte à des films anhydres hydrosolubles et à leurs utilisations dans le domaine cosmétique ou dermatologique, notamment en dissolution dans une composition appropriée pour une application topique.

L'invention se rapporte aussi à un kit comprenant le dit film et une composition aqueuse dans laquelle est dissoute ce film.

L'invention se rapporte encore à une utilisation cosmétique du mélange obtenu à partir du film et de la composition, pour le traitement cosmétique de la peau, des muqueuses, des phanères ou des cheveux.

A côté des produits cosmétiques habituels se présentant sous forme liquide, gélifiée ou solide, il est connu d'utiliser des préparations cosmétiques sous forme de films fins hydrosolubles, comme décrit par exemple dans le document JP-A-2002/212027, ou dans les documents US-A-2004/0071755, WO-A-2002/05789, US-A-2002/0127254 et WO-A-2003/075812 qui décrivent l'obtention de films polymériques anhydres pour une administration directe de compositions cosmétiques sur la peau préalablement mouillée. De plus, le document US-A-2003/0186826 décrit une composition cosmétique sèche à base de polymères et de tensioactifs, à administrer sur la peau ou les cheveux avec de l'eau.

Par ailleurs, il est connu par le document FR-A-2,868,949, des kits comportant un ou plusieurs films fins anhydres et une composition fluide dans laquelle sont introduits ce ou ces films. Au moment de l'application, le ou les films fins anhydres sont dissous extemporanément avec la composition fluide pour former une nouvelle composition à appliquer sur la peau, les muqueuses ou les phanères. Une telle composition cosmétique permet de s'affranchir des problèmes de stabilité colloïdale ou chimique, de moduler la rhéologie finale de la préparation et sa concentration en actifs, ou encore de proposer des formulations « à la demande ». En outre, cela permet d'associer des molécules incompatibles entre elles dans une même composition, ce qui est une solution plus économique que l'application de deux compositions successives. Par ailleurs, cela permet de pouvoir déposer sur la peau des doses d'actifs significativement plus élevées qu'avec une formulation conventionnelle.

En outre, le document FR-A-2,871,685 décrit un kit de rasage comportant un film anhydre soluble dans un solvant tel que l'eau.

Toutefois, ces films anhydres présentent l'inconvénient d'avoir des propriétés de dissolution imparfaites, c'est-à-dire que la dissolution de ces films est incomplète dans un temps raisonnable (on entend ici par « temps raisonnable », un temps inférieur à 30 secondes). Or, pour avoir une composition finale satisfaisante, il est nécessaire que la dissolution du film soit complète en un temps raisonnable pour que l'utilisateur n'ait pas à attendre pour appliquer le produit. En outre, la dissolution incomplète du film se traduit par l'apparition d'amas particulièrement inesthétiques lors de la dissolution du film et lors de son application sur la peau ou sur toute autre matière kératiinique.

Il subsiste donc le besoin de disposer de films anhydres ayant une cinétique de dissolution dans des formulations fluides, qui soit rapide et qui permette une utilisation optimisée de tels films.

La demanderesse a trouvé de manière surprenante que l'addition de polysiloxanes oxyalkylénés particuliers dans ces films fins permettait d'atteindre le but recherché.

La présente invention a donc pour objet un film anhydre hydrosoluble comportant (i) au moins un polymère filmogène hydrosoluble ou hydrodispersible, (ii) au moins un agent épaississant polysaccharidique, (iii) au moins un polysiloxane oxyalkyléné hydrosoluble ou hydrodispersible, et (iv) au moins un plastifiant choisi parmi les polyols.

L'invention a aussi pour objet un produit cosmétique obtenu par mélange extemporané d'au moins un film tel que défini ci-dessus, avec une quantité appropriée d' une composition aqueuse.

L'invention a aussi pour objet un kit de formulation d'un produit cosmétique comprenant :
i) une composition aqueuse ; et
ii) au moins un film anhydre hydrosoluble tel que défini ci-dessus.

Ledit produit cosmétique est obtenu en ajoutant à la composition aqueuse, un ou plusieurs films. Les films ajoutés peuvent avoir une composition identique ou différente.

L'invention a encore pour objet un kit de formulation customisée d'un produit cosmétique, notamment de soin ou de maquillage, comprenant :
i) une composition aqueuse ;
ii) une pluralité de films anhydres hydrosolubles, identiques ou différents, destiné(s) à être mélangé(s) à la composition aqueuse pour former ledit produit cosmétique, et
iii) éventuellement des instructions, notamment sur une notice explicative, pour formuler à façon ledit produit cosmétique en fonction du nombre de films anhydres hydrosolubles identiques ou différents à mélanger à la composition.

On entend par "formulation customisée", une formulation adaptée à la demande du consommateur au moment de l'utilisation.

Le produit cosmétique final est obtenu par mélange extemporané d'un ou plusieurs films anhydres hydrosolubles et d'une quantité appropriée de la composition aqueuse. On entend par "quantité appropriée" de composition aqueuse, une quantité telle que le ou les films s'y dissolvent rapidement. Cette quantité peut aller par exemple de 10 à 1000 mg, de préférence de 50 à 800 mg et mieux de 100 à 500 mg. La dose appropriée de composition aqueuse peut être obtenue en utilisant des formes de présentation en mono-dose, telles que des sachets, des tubes, des ampoules, des seringues pré-remplies, des capsules molles, des coques ou barquettes en plastique thermoformé. Une dose appropriée peut également être obtenue à partir d'une présentation multi-doses en utilisant un système distribuant une dose pré-définie. Un tel système peut être un flacon pompe, un aérosol, une pipette ou une seringue graduée, un compte-gouttes.

D'autres objets encore apparaîtront dans la description détaillée qui suit.

### A. Films anhydres hydrosolubles

Par "film", on entend dans la présente demande, un solide fin. On entend par "fin", un solide ayant une épaisseur d'au maximum 1000 µm.

Ce film est préhensible, c'est-à-dire qu'il a généralement une dimension adéquate pour pouvoir être facilement manipulé par l'utilisateur. Il peut avoir une forme de carré, de rectangle, de disque ou toute autre forme. Chaque film a généralement une épaisseur de 10 µm à 1000 µm, de préférence de 20 à 500 µm et mieux de 50 à 300 µm. Il peut avoir une surface de 0,25 à 25 cm² et de préférence de 2 à 10 cm².

Par ailleurs, par "film anhydre", on entend dans la présente demande, un film contenant moins de 15 % en poids d'eau (0 à 15 % en poids d'eau), de préférence moins de 10 % en poids (0 à 10 % en poids d'eau) et mieux moins de 5 % en poids (0 à 5 % en poids d'eau) par rapport au poids total du film, et de préférence encore, ne contenant pas d'eau.

En outre, on entend dans la présente demande par « film hydrosoluble », un film qui se dissout dans l'eau.

Le film qui est utilisé à des fins cosmétiques ou dermatologiques contient un milieu physiologiquement acceptable, c'est-à-dire compatible avec la peau, les muqueuses, les cheveux et le cuir chevelu.

### Polysiloxanes oxyalkylénés

Les polysiloxanes oxyalkylénés utilisés selon l'invention sont hydrosolubles ou hydrodispersibles. On entend par "hydrosolubles ou hydrodispersibles" des polysiloxanes ayant une solubilité dans l'eau mesurée à 25°C au moins égale à 0,1 gramme/litre (g/l) (obtention d'une solution macroscopiquement isotrope et transparente, colorée ou non). Cette solubilité est de préférence supérieure ou égale à 1 g/l.

Ces polysiloxanes sont choisis de préférence parmi les silicones hydrosolubles comportant au moins un groupement polyoxyalkyléné monovalent terminal ou pendant, et qui, introduits à 0,05% en poids dans une solution aqueuse, sont susceptibles de réduire la tension superficielle de l'eau à une valeur inférieure à 35 mN/m, et de préférence inférieure à 30 mN/m.

Les polysiloxanes peuvent être en particulier des polydiméthylsiloxanes ou PDMS.

Les polysiloxanes oxyalkylénés conformes à l'invention sont choisis plus préférentiellement parmi les silicones hydrosolubles de formule générale (a) suivante :

R²₃SiO(R²₂SiO)ₚ(R²PESiO)_{q}SIR²₃ (a)

dans laquelle
- les radicaux R², identiques ou différents, désignent un radical hydrocarboné monovalent choisi parmi les radicaux alkyles, aryles et aralkyles ayant au plus 10 atomes de carbone ;
- p varie de 0 à 150, de préférence de 0 à 100 et plus préférentiellement de 0 à 30 ;
- q varie de 1 à 12, de préférence de 1 à 10 et plus préférentiellement de 1 à 8 ;
- le groupe polyéther PE a la formule (b) suivante

   -CₓH₂ₓ(OC₂H₄)_{y}(OC₃H₆)_{z}OR³ (b)
dans laquelle :
- x varie de 1 à 8 et de préférence varie de 2 à 4 et plus préférentiellement est égal à 3 ;
- y est supérieur à 0,
- z est supérieur ou égal à 0 ; les valeurs de y et z étant tels que le poids moléculaire total de la portion polyoxyalkylénée du groupe polyéther PE varie de 200 à 10.000 et plus préférentiellement de 350 à 4000 ;
- R³ désigne l'hydrogène, un groupe alkyle en C₁-C₈ ou un groupe acyle en C₂-C₈.

Il est à noter que lorsque z est différent de 0, les unités polyoxyéthylène et polyoxypropylène peuvent être distribuées de manière aléatoire le long de la chaîne polyéther PE ou répartis en blocs ou bien à la fois répartis en blocs et de manière aléatoire.

De préférence, les radicaux R² sont choisis parmi les groupes alkyles en C₁-C₄ et les groupes hexyle, phényle et benzyle. Et plus particulièrement, les radicaux R² sont choisis parmi les groupes alkyles tels que méthyle, éthyle, butyle, encore plus particulièrement ils désignent le radical méthyle.

De préférence, les radicaux R³ sont choisis parmi les groupes alkyles en C₁-C₄ et encore plus particulièrement désignent le radical méthyle.

Les silicones hydrosolubles de formule (a) peuvent être obtenues selon le procédé décrit dans le document US-A-4,847398.

Parmi les silicones hydrosolubles de formule (a), on utilise de préférence celles de formule (a') suivante :

MeSiO(MeSiO)ₚ(MePESiO)_{q}SIMe₃ (a')

dans laquelle p et q ont les mêmes valeurs qu'indiquées ci-dessus pour la formule (a), et Me désigne le radical méthyle ; PE désigne :

-(CH₂)₃O(OC₂H₄)_{y}(OC₃H₆)_{z}OR³ (b')

où y et z ont les mêmes valeurs qu'indiquées ci-dessus pour la formule b, et R³ désigne hydrogène ou un groupe alkyle en C₁-C₄. et plus particulièrement le radical méthyle

Comme autre famille de polysiloxanes hydrosolubles utilisables selon l'invention, on peut citer les silicones ramifiées de formule (c) suivante :

(MeSiO)_{q-2}[SiOMe2)_{p/q} OPE]_{q} (c)

où p et q ont les mêmes valeurs qu'indiquées ci-dessus dans la formule (a) ; Me signifie méthyle ;PE désigne le groupe de formule (d) suivante :

-(OC₂H₄)_{y}(OC₃H₆)_{z}R³ (d)

où y et z ont les mêmes valeurs indiquées ci-dessus dans la formule (b) et R³ désigne un groupe alkyle en C₁-C₄. et plus particulièrement le radical méthyle.

On peut bien sûr utiliser un mélange des silicones de formule (a) et de formule (c).

De tels polysiloxanes oxyalkylénés sont par exemple vendus par la société OSI sous les dénominations commerciales Silwet L-7210^{®} (nom INCI : Dimethicone Copolyol), Silwet L-7220^{®} (nom INCI : Dimethicone Copolyol) ; Silwet L-7002^{®}, Silwet L-7600^{®} (nom INCI : Dimethicone Copolyol), Silwet L-7604^{®} (nom INCI : PEG-8 Dimethicone), Silwet L-7605^{®} , Silwet L-7607^{®}, Silwet 1614, Silwet L-7657^{®} , Silwet L-7200^{®} (nom INCI : Dimethicone Copolyol), Silwet L-7230^{®}, Silsoft 305^{®} (nom INCI : Dimethicone Copolyol), Silsoft 820 (nom INCI: Dimethicone Copolyol), Silsoft 880^{®} (nom INCI : PEG-12 dimethicone), Tego wet 260^{®}, Tego wet 500^{®}, Tego wet 505^{®} et Tego wet 510^{®}.

Le tableau suivant rassemble des valeurs de tensions superficielles à 25°C de solutions aqueuses comprenant 0,05% (en poids) de différents polysiloxanes oxyalkylénés :

| Polysiloxane oxyalkyléné | Tension superficielle à 0,05% dans l'eau (mN/m) |
|---|---|
| Tegowet 500 | 33 |
| Tegowet 510 | 29 |
| Silsoft 880 | 26 |
| Silsoft 305 | 21 |

La quantité de polysiloxane(s) oxyalkyléné(s) dans le film selon l'invention peut aller par exemple de 0,5 à 30 % en poids, de préférence de 1 à 20 % en poids par rapport au poids total du film.

### Polymères filmogènes

Le film contient un ou de plusieurs polymères filmogènes hydrosolubles ou hydrodispersibles. On entend par "hydrosolubles ou hydrodispersibles" des polymères ayant une solubilité dans l'eau mesurée à 25°C au moins égale à 0,1 gramme/litre (g/l) (obtention d'une solution macroscopiquement isotrope et transparente, colorée ou non). Cette solubilité est de préférence supérieure ou égale à 1 g/l. Comme les autres composés, ces polymères doivent être physiologiquement acceptables c'est-à-dire compatibles avec la peau, les muqueuses, les cheveux et le cuir chevelu.

Par « polymère filmogène », on entend un polymère apte à former à lui seul ou en présence d'un agent auxiliaire de filmification, un film continu, et de préférence un film dont la cohésion et les propriétés mécaniques sont telles que ledit film peut être isolé d'un support.

Ces polymères sont catalogués sous la rubrique « Film Formers » dans le dictionnaire cosmétique « International Cosmetic Ingredient dictionary and Handbook ». (voir par exemple pages 2903 à 2906 de la neuvième édition - 2002).

Les polymères filmogènes peuvent être choisis par exemple parmi :
- les polymères vinyliques tels que l'acétate de polyvinyle, les polyvinylpyrrolidones, les copolymères du méthylvinyléther et de l'anhydride maléique, le copolymère de l'acétate de vinyle et de l'acide crotonique, les copolymères de la vinylpyrrolidone et de l'acétate de vinyle, les copolymères de la vinylpyrrolidone et du caprolactame, les alcools polyvinyliques ;
- les dérivés cellulosiques filmogènes, comme l'hydroxyéthylcellulose, l'hydroxypropylcellulose, l'hydroxypropylméthylcellulose, la méthylcellulose, l'éthylhydroxyéthylcellulose, la carboxyméthylcellulose, et les dérivés quaternisés de la cellulose ;
- les amidons et leurs dérivés ;
- les polymères d'origine naturelle, éventuellement modifiés, tels que le pullulane, la pectine, la mannane, les galactomannanes, les glucomannanes et leurs dérivés, la gomme arabique, la gomme de guar, la gomme de xanthane, la gomme de karaya ; les alginates, les carraghénanes, les ulvanes et autres colloïdes algaux ; l'acide hyaluronique et ses dérivés ; la gomme laque, la gomme sandaraque, les dammars, l'élémis, les copals ; l'acide désoxyribonucléique ; les mucopolysaccharides tels que l'acide hyaluronique, le sulfate de chondroïtine ;
- les polymères dérivant de la chitine ou du chitosane, anioniques, cationiques, amphotères ou non ioniques,
- les polymères protéiques, tels que les protéines de blé ou de soja ; la kératine et ses dérivés, par exemple les hydrolysats de kératine et les kératines sulfoniques ; la caséine ; l'albumine ; le collagène ; la gluteline ; le glucagon ; le gluten ; la zéine ; les gélatines et leurs dérivés ;
- les copolymères acryliques de phosphoryle choline, tels que le poly-2-(methacryloyloxyethyl) phosphorylcholine commercialisé sous la dénomination Lipidure HM par la société NOF Corporation (nom INCI : Polyphosphorylcholine glycol acrylate) ;
- les complexes anion-cation de type gomme arabique / gélatine ou gomme arabique /chitosane, ou collagène / GlycosAminoGlycane
- et les mélanges de ces polymères.

Selon un mode préféré de réalisation de l'invention, le polymère filmogène est choisi parmi les polymères vinyliques, les dérivés cellulosiques et leurs mélanges.

De manière préférée, le polymère vinylique est l'acétate de polyvinyle (PVA), qui est préparé par polymérisation radicalaire du monomère acétate de vinyle puis hydrolyse. On peut utiliser notamment l'acétate de polyvinyle hydrolysé à 88%, tel que celui vendu sous la dénomination CELVOL 540 PV ALCOHOL par la société Celanese Chemicals.

De manière préférée, les dérivés cellulosiques sont choisis parmi l'hydroxypropylcellulose (HPC) et l'hydroxypropylméthylcellulose (HPMC). Ces polymères sont solubles dans l'eau ainsi que dans des solvants organiques. Ceci permet d'accroître le champ de solubilité des films les contenant. Le choix du poids moléculaire de ces polymères cellulosiques doit être fait de manière judicieuse pour augmenter la dissolution des films.

Les HPC utilisés de façon préférée sont ceux commercialisée par la société Hercules sous la dénomination :
- Klucel® MF dont le poids moléculaire est 850 000 (viscosité 4000-6500 mPa à 2% dans l'eau)
- Klucel® EF dont le poids moléculaire est 80 000 (viscosité 300-600 mPa à 10% dans l'eau)

L'HPMC utilisée de façon préférée est l'hydroxypropylméthylcellulose de viscosité 40-60 cps à 2% dans l'eau à 20°C, commercialisée par la société Sigma-Aldrich.

De manière préférée, le polymère filmogène est choisi parmi l'acétate de polyvinyle, l'hydroxypropylcellulose, l'hydroxypropylméthylcellulose, et leurs mélanges.

La quantité de polymère(s) filmogène(s) hydrosoluble(s) dans le film selon l'invention peut aller par exemple de 10 à 95 % en poids, en particulier de 20 à 70 % en poids, et plus particulièrement de 30 à 60 % en poids par rapport au poids total dudit film.

On peut utiliser dans le film de l'invention, un polymère qui soit à la fois un polymère filmogène et un polymère épaississant, choisi par exemple parmi les dérivés cellulosiques et les polymères d'origine naturelle qui peuvent être à la fois filmogènes et épaississants. La quantité d'utilisation reste celle indiquée ci-dessus : par exemple de 10 à 95 % en poids, en particulier de 20 à 70 % en poids, et plus particulièrement de 30 à 60 % en poids par rapport au poids total dut film.

### Agent épaississant polysaccharidique

Le film selon l'invention contient au moins un agent épaississant polysaccharidique.

Les agents épaississants polysaccharidiques utilisés dans le film selon l'invention sont choisis parmi les polysaccharides à pouvoir gélifiant. On définit par « pouvoir gélifiant » le fait qu'à une concentration supérieure ou égale à 0,5 % en poids dans l'eau, la viscosité des solutions ainsi obtenues est supérieure ou égale à 0,01 Pa.s pour un taux de cisaillement égale à 1 s⁻¹, les mesures étant réalisées à 25°C à l'aide d'un rhéomètre RheoStress RS150 de Haake en configuration cône - plan, les mensurations du cône de mesure étant les suivantes : diamètre : 60 mm et angle : 2°.

Les agents épaississants polysaccharidiques peuvent être choisis notamment parmi la gomme arabique, la gomme de ghatti, la gomme de karaya, la gomme de caroube, la gomme de guar, la gomme de tamarin, la gomme de xanthane, la gellane, les pectines, le tragacanth, l'agar, les alginates, le carrageenan, le furcelleran, le konjac et les dérivés de cellulose, et leurs mélanges.

Selon un mode préféré de réalisation de l'invention, les agents épaississants polysaccharidiques sont choisis parmi les carraghénanes qui sont des polysaccharides linéaires extraits de certaines algues rouges de la famille des Rhodophycée. Ils sont constitués de résidus β-1,3 et α-1,4 galactoses en alternance, de nombreux résidus galactoses pouvant être sulfatés. Il existe trois types de carraghénanes, appelés Kappa-carraghénane, lota-carraghénane et Lambda-carraghénane. Cette famille de polysaccharides est décrite par exemple dans le chapitre 3 du livre « Food Gels » édité par Peter HARRIS, Elsevier 1989.

On peut en particulier utiliser le carraghénane vendu sous la dénomination SATIAGUM UTC 10 par la société Degussa.

La quantité d'agent(s) épaississant(s) dans le film selon l'invention peut aller par exemple de 0,5 à 40 % en poids, en particulier de 1 à 20 % en poids, et plus particulièrement de 5 à 10 % en poids par rapport au poids total du film.

### Plastifiant

Le film comprend en outre un ou plusieurs plastifiants choisis parmi les polyols tels que la glycérine, le sorbitol, les mono- et/ou di-saccharides, le dipropylène glycol, le butylène glycol, le pentylène glycol, les polyéthylène glycols tels que le PEG-400. Il peut contenir en outre un ou plusieurs plastifiants autres que les polyols. La quantité de plastifiant(s) peut aller par exemple de 1 à 40 % en poids et mieux de 2 à 15 % en poids par rapport au poids total du film.

Les films peuvent être conditionnés dans un article facilitant leur préhension, tel que celui décrit dans le document FR-A-2,863,167 dont le contenu est inclus dans la présente demande par référence. Les films peuvent notamment être conditionnés dans une boîte plastique distributrice, dans un sachet individuel ou dans un blister. Les films peuvent être conditionnés dans un boîtier de type à tiroir ou à couvercle articulé sur un fond, le dit boîtier pouvant comporter des moyens destinés à faciliter la distribution des articles. Les moyens de distribution peuvent être par exemple du type de ceux décrits par exemple dans les documents US-A-2,973,882, GB-A-2,358,627, CH-A-461025, ou US-A-6,578,732.

### Composition aqueuse

On entend par "composition aqueuse" dans la présente demande, une composition contenant au moins de l'eau. La quantité d'eau est variable selon la galénique de la composition mais elle est généralement d'au moins 20 % en poids par rapport au poids total de la composition.

La composition aqueuse, outre l'eau, peut contenir un solvant organique soluble dans l'eau, choisi par exemple parmi les mono-alcools inférieurs comportant de 1 à 8 atomes de carbone et en particulier 1 à 6 atomes de carbone, comme l'éthanol, l'isopropanol, le propanol, le butanol ; les polyéthylène glycols ayant de 6 à 80 oxydes d'éthylène ; les polyols comme le propylène glycol, l'isoprène glycol, le butylène glycol, la glycérine, le sorbitol ; l'acétone ; et leurs mélanges.

Il s'agit d'une composition plus ou moins fluide par opposition à une composition solide. On entend par "composition plus ou moins fluide" dans la présente demande, une composition dont on peut mesurer la viscosité, et qui va du liquide au semi-solide (crème ou pâte molle). La viscosité peut aller par exemple de 1 à 20000 mPa.s (1 à 20000 cPoises), de préférence de 1 à 15000 mPa.s, cette viscosité étant mesurée à 25°C à l'aide du Rheomat RM180 de Rheometric Scientific, cet appareil étant équipé d'un mobile différent selon les viscosités.

Cette composition comporte un milieu physiologiquement acceptable, c'est-à-dire compatible avec la peau, les muqueuses, les cheveux et le cuir chevelu.

La composition aqueuse peut contenir aussi des corps gras. On entend par "corps gras" tout composé insoluble dans l'eau, ce composé pouvant être une huile (corps gras liquide) ou un corps gras solide. La composition se présente alors sous forme de dispersion ou d'émulsion.

La composition aqueuse peut donc se présenter sous forme de solutions, d'émulsions plus ou moins fluides (lotions ou crèmes selon la viscosité), par exemple d'émulsions eau-dans-huile (E/H) ou huile-dans-eau (H/E) ou multiple (E/H/E ou H/E/H), choisies notamment parmi les émulsions classiques ou les émulsions particulières comme par exemple parmi :
- les émulsions H/E à base de globules huileux pourvus d'un enrobage cristal liquide lamellaire, telles que décrites dans les documents EP-A-641557 et EP-A-705593 ;
- Les émulsions H/E sans tensioactif, stabilisées par des polymères anioniques hydrodispersibles, telles que celles décrites dans le document EP-A-864320 ;
- Les émulsions H/E à base de polymères dérivés d'acide 2-acrylamido 2-méthylpropane sulfonique (Polymère AMPS), telles que décrites dans le document EP-A-815844 ;
- Les émulsions H/E stabilisées par des polymères d'AMPS hydrophobes, telles décrites dans les documents EP-A-1,069,142, WO-A-2002/43689, WO-A-2002/44231, WO-A-2002/44271, WO-A-2002/44270, WO-A-2002/43686, WO-A-2002/44267, WO-A-2002/43688, WO-A-2002/43677, WO-A-2002/43687, WO-A-2002/44230 ;
- Les émulsions fluides à base de polymères thermoassociatifs, telles que décrites dans les documents EP-A-1,355,990, EP-A-1,355,625, EP-A-1,307,501, EP-A-1,363,964 ;
- Les émulsions H/E obtenues par la méthode PIT (émulsion obtenue par inversion de phase. PIT = Phase Inversion Temperature), telles que décrites dans les documents WO-A-89/11907, DE-A-4318171, et EP-A-815846 ;
- Les nanoémulsions telles que celles décrites dans les demandes EP-A-728460, EP-A-780114, EP-A-780115, EP-A-879589, EP-A-1,010,413, EP-A-1,010,414, EP-A-1,010,415, EP-A-1,010,416, EP-A-1,013,338, EP-A-1,016,453, EP-A-1,018,363, EP-A-1,020,219, EP-A-1,025,898, EP-A-1,120,102, EP-A-1,120,101, EP-A-1,160,005, EP-A-1,172,077 et EP-A-1,353,629.

La composition aqueuse peut être aussi sous forme de produits aqueux moussants, tels que décrits par exemple dans les documents EP-A-1,166,747, EP-A-1,172,096, EP-A-1,172,095, EP-A-1,174,122, EP-A-1,277,463, EP-A-1,295,594, FR-A-2,824,262.

Selon un mode particulier de réalisation de l'invention, la composition aqueuse utilisée selon l'invention est une émulsion. La proportion de la phase huileuse de l'émulsion peut aller de 1 à 80 % en poids, et de préférence de 1 à 50 % en poids par rapport au poids total de la composition. Les huiles, et les émulsionnants et co-émulsionnants éventuellement présents, utilisés dans la composition sous forme d'émulsion sont choisis parmi ceux classiquement utilisés dans le domaine cosmétique ou dermatologique. L'émulsionnant et le co-émulsionnant quand ils sont présents, le sont généralement, en une proportion allant de 0,2 à 30 % en poids, de préférence de 0,3 à 20 % en poids et mieux de 0,5 à 15 % en poids par rapport au poids total de la composition. L'émulsion peut en outre, contenir des vésicules lipidiques.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant la phase continue de l'émulsion à obtenir (E/H ou H/E). Quand l'émulsion est multiple, elle comporte généralement un émulsionnant dans l'émulsion primaire et un émulsionnant dans la phase externe dans laquelle est introduite l'émulsion primaire.

Comme émulsionnants utilisables pour la préparation des émulsions E/H, on peut citer par exemple les alkyl esters ou éthers de sorbitan, de glycérol ou de sucres ; les tensioactifs siliconés comme les dimethicone copolyols tels que le mélange de cyclomethicone et de dimethicone copolyol, vendu sous les dénominations DC 5225 C et DC 3225 C par la société Dow Corning, et comme les alkyl-dimethicone copolyols tels que le Laurylmethicone copolyol vendu sous la dénomination "Dow Corning 5200 Formulation Aid" par la société Dow Corning, le Cetyl dimethicone copolyol vendu sous la dénomination Abil EM 90^{R} par la société Goldschmidt et le mélange de Polyglyceryl-4 isostearate/Cetyl dimethicone copolyol/Hexyl laurate vendu sous la dénomination Abil WE 09^{R} par la société Goldschmidt. On peut y ajouter aussi un ou plusieurs co-émulsionnants, qui, de manière avantageuse, peuvent être choisis dans le groupe comprenant les esters d'acide gras à chaîne ramifiée et de polyol, et notamment les esters d'acide gras à chaîne ramifiée et de glycérol et/ou de sorbitan et par exemple l'isostéarate de polyglycéryle, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

Comme émulsionnants utilisables pour la préparation des émulsions H/E, on peut citer par exemple les émulsionnants non ioniques tels que les esters d'acides gras et de polyols oxyalkylénés (plus particulièrement polyoxyéthylénés), et par exemple les stéarates de polyéthylène glycol comme le stéarate de PEG-100, le stéarate de PEG-50 et le stéarate de PEG-40 ; et leurs mélanges tels que le mélange de mono-stéarate de glycéryle et de stéarate de polyéthylène glycol (100 OE) commercialisé sous la dénomination SIMULSOL 165 par la société SEPPIC ; les esters d'acides gras et de sorbitan oxyalkylénés comprenant par exemple de 20 à 100 OE, et par exemple ceux commercialisés sous les dénominations commerciales Tween 20 ou Tween 60 par la société Uniqema ; les éthers d'alcools gras oxyalkylénés (oxyéthylénés et/ou oxypropylénés) ; les esters de sucres, alcoxylé ou non, comme le stéarate de sucrose et comme le PEG-20 méthylglucose sesquistéarate ; les esters de sorbitan tels que le palmitate de sorbitan commercialisé sous la dénomination Span 40 par la société Uniqema ; les esters de diacide et d'alcool gras, tels que le tartrate de dimyristyle ; les mélanges de ces émulsionnants comme par exemple le mélange de stéarate de glycéryle et de stéarate de PEG-100, commercialisé sous la dénomination Arlacel 165 par la société Uniqema ; et les mélanges contenant ces émulsionnants, tels que le mélange de tartrate de dimyristyle, d'alcool cétéarylique, de Pareth-7 et de PEG-25 laureth-25, commercialisé sous la dénomination Cosmacol PSE par la société Sasol (nom INCI : Dimyristyl tartrate / cetearyl alcool /12-15 Pareth 7 / PPG 25 laureth 25).

On peut ajouter à ces émulsionnants, des co-émulsionnants tels que par exemple les alcools gras ayant de 8 à 26 atomes de carbone, comme l'alcool cétylique, l'alcool stéarylique et leur mélange (alcool cétéarylique), l'octyl dodécanol, le 2-butyloctanol, le 2-hexyldécanol, le 2-undécylpentadécanol ou l'alcool oléique, ou les acides gras.

On peut aussi préparer des émulsions sans tensioactifs émulsionnants ou en contenant moins de 0,5 % du poids total de la composition, en utilisant des composés appropriés, par exemple les polymères ayant des propriétés émulsionnantes tels que les polymères commercialisés sous les dénominations CARBOPOL 1342 et PEMULEN par la société Noveon ; ou les polymères en émulsion tels que celui commercialisé sous la dénomination Sepigel 305 par la société Seppic (nom INCI : Polyacrylamide / C13-C14 isoparaffine / laureth-7) ; les particules de polymères ioniques ou non ioniques, plus particulièrement des particules de polymère anionique comme notamment les polymères d'acide isophtalique ou d'acide sulfoisophtalique, et en particulier les copolymères de phtalate / sulfoisophtalate / glycol (par exemple diéthylèneglycol / Phtalate / isophtalate/1,4-cyclohexane-diméthanol (nom INCI : Diglycol/CHDM/Isophtalates/ SIP Copolymer) vendus sous les dénominations Eastman AQ polymer (AQ35S, AQ38S, AQ55S, AQ48 Ultra) par la société Eastman Chemical. On peut aussi préparer des émulsions sans émulsionnants, stabilisées par des particules siliconées ou des particules d'oxyde métallique tels que TiO2 ou autres.

Quand la composition aqueuse est sous forme d'émulsion, elle comporte au moins une phase huileuse qui contient au moins une huile, notamment une huile cosmétique. On entend par "huile" un corps gras liquide à la température ambiante (25°C).

Comme huiles utilisables dans la composition de l'invention, on peut citer par exemple :
- les huiles hydrocarbonées d'origine animale, telles que le perhydrosqualène (ou squalane) ;
- les huiles hydrocarbonées d'origine végétale, telles que les triglycérides liquides d'acides gras comportant de 4 à 10 atomes de carbone comme les triglycérides des acides heptanoïque ou octanoïque ou encore les huiles d'origine végétale, par exemple les huiles de tournesol, de maïs, de soja, de courge, de pépins de raisin, de sésame, de noisette, d'abricot, de macadamia, d'arara, de coriandre, de ricin, d'avocat, l'huile de jojoba, l'huile de beurre de karité, ou encore les triglycérides des acides caprylique/caprique comme ceux vendus par la société Stearineries Dubois ou ceux vendus sous les dénominations Miglyol 810, 812 et 818 par la société Dynamit Nobel ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; les esters du pentaérythritol comme le tétraisostéarate de pentaérythrytyle ; les dérivés lipophiles d'acides aminés, tels que le lauroyl sarcosinate d'isopropyle (nom INCI : Isopropyl Lauroyl sarcosinate) commercialisé sous la dénomination Eldew SL 205 par la société Ajinomoto ;
- les hydrocarbures linéaires ou ramifiés, d'origines minérale ou synthétique, tels que les huiles minérales (mélange d'huiles hydrocarbonées dérivées du pétrole ; nom INCI : Mineral oil), les huiles de paraffine, volatiles ou non, et leurs dérivés, la vaseline, les polydécènes, l'isohexadécane, l'isododécane, l'isoparaffine hydrogéné tel que l'huile de Parléam® commercialisée par la société NOF Corporation (nom INCI ; Hydrogenated Polyisobutene) ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclopentasiloxane et la cyclohexadiméthylsiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- les huiles fluorées telles que celles partiellement hydrocarbonées et/ou siliconées comme celles décrites dans le document JP-A-2-295912 ;
- les éthers tels que l'éther dicaprylique (nom INCI : Dicaprylyl ether) ; et les benzoates d'alcools gras en C₁₂-C₁₅ (Finsolv TN de FINETEX) ;
- leurs mélanges.
   La composition aqueuse et/ou le ou les films peuvent contenir en outre tout composé additif approprié, comme par exemple les adjuvants de formulation et/ou les actifs.

### Actifs

Le film anhydre hydrosoluble ou la composition aqueuse ou les deux peuvent contenir au moins un actif. Un des intérêts de l'association selon l'invention comprenant le ou les films et la composition aqueuse est par exemple de pouvoir obtenir un produit cosmétique contenant des actifs incompatibles, un ou plusieurs actifs compatibles étant présents dans la composition aqueuse et un ou plusieurs actifs différents et incompatibles avec ceux de la composition aqueuse étant présents dans le ou les films anhydres. Le mélange extemporané de la composition aqueuse et du ou des films aboutit alors à un produit contenant des actifs incompatibles, leur incompatibilité ne présentant pas d'inconvénient puisque l'utilisation du produit est immédiate. Cela peut permettre aussi de pouvoir obtenir des produits ayant des concentrations en actifs plus élevées que les concentrations que l'on pourrait obtenir en utilisant une seule composition.

On entend par « actif » tout composé ayant un effet bénéfique sur la matière kératinique sur laquelle est appliqué le produit final, notamment sur la peau.

A titre d'exemples et sans que cette liste soit limitative, on peut citer comme actifs ayant une application cosmétique ou dermatologique

I) les actifs hydratants, tels que par exemple le lactate de sodium ; les polyols, et en particulier la glycérine, le sorbitol, les polyéthylène glycols ; le mannitol ; les acides aminés ; l'acide hyaluronique ; la lanoline ; l'urée et les mélanges contenant de l'urée, tels que le NMF (« Natural Moisturising Factor ») ; les dérivés d'urée, notamment les hydroxyalkyl urées telles que l'hydroxyéthyl urée disponible dans le commerce, sous forme de mélange à 50% en poids dans l'eau, auprès de la société NATIONAL STARCH sous la dénomination commerciale Hydrovance^{®} ; la vaseline ; les sels et dérivés d"acide pyrrolidone carboxylique, tels que l'acide N-lauroyl pyrrolidone carboxylique ; les acides gras essentiels ; les huiles essentielles ; et leurs mélanges.

II) les actifs anti-vieillissement qui peuvent être choisis parmi tous les actifs susceptibles de traiter ou de prévenir tout signe de vieillissement de la peau. Ils peuvent être choisis par exemple parmi les agents anti-radicaux libres, les agents kératolytiques, les vitamines, les agents anti-élastase et anti-collagénase, les protides, les dérivés d'acides gras, les stéroïdes, les oligo-éléments, les extraits d'algues et de planctons, les enzymes et co-enzymes, les flavonoïdes, les céramides, les agents myorelaxants, les glycosides aptes à stimuler la synthèse des glycosaminoglycannes, et leurs mélanges.
1) Comme agents anti-radicaux libres et anti-oxydants, on peut citer notamment les dérivés de l'acide phosphonique tels que l'acide éthylène diamine tétra(méthylène phosphonique), l'acide hexaméthylène diamine tétra(méthylène phosphonique), l'acide diéthylène triamine penta(méthylènephosphonique), et leurs sels et en particulier leurs sels de sodium ; l'acide éthylène diamine tétracétique et ses sels tels que le sel de sodium ; la guanosine ; la superoxydismutase ; le tocophérol (vitamine E) et ses dérivés (acétate) ; l'éthoxyquine ; la lactoferrine ; la lactoperoxydase, et les dérivés nitroxydes ; le glutathion peroxydase ; les extraits végétaux à activité antiradicalaire tels que l'extrait aqueux de germe de blé commercialisé par la société Silab sous la référence Detoxiline ; le thé vert ; et leurs mélanges.
2) Comme agents kératolytiques, on peut citer par exemple les α-hydroxy-acides notamment les acides dérivés de fruits, comme les acides glycolique, lactique, malique, citrique, tartrique, mandélique, leurs dérivés et leurs mélanges ; les β-hydroxy-acides comme l'acide salicylique et ses dérivés tels que l'acide n-octanoyl-5-salicylique ou l'acide n-dodécanoyl-5-salicylique ; les α-céto-acides comme l'acide ascorbique ou vitamine C et ses dérivés tels que ses sels comme l'ascorbate de sodium, l'ascorbylphosphate de magnésium ou de sodium ; ses esters comme l'acétate d'ascorbyle, le palmitate d'ascorbyle et le propionate d'ascorbyle, ou ses sucres comme l'acide ascorbique glycosylé, et leurs mélanges ; les β-céto-acides ; les rétinoïdes comme le rétinol (vitamine A) et ses esters, le rétinal, l'acide rétinoïque et ses dérivés, ainsi que les rétinoïdes décrits dans les documents FR-A-2,570,377, EP-A-199636, EP-A-325540, EP-A-402072 ; l'adapalène ; les caroténoïdes ; et leurs mélanges.
3) Comme vitamines, outre les vitamines A, E et C indiquées ci-dessus, on peut citer en particulier la vitamine B3 (ou vitamine PP ou niacinamide) et ses dérivés (nicotinate de tocophérol, esters d'alcool nicotinyle et d'acides carboxyliques, 2-chloronicotinamide, 6-méthylnicotinamide, 6-aminonicotinamide, N-méthylnicotinamide, N,N-diméthylnicotinamide, N-(hydroxyméthyl)-nicotinamide, imide d'acide quinolinique, nicotinanilide, N-benzylnicotinamide, N-éthylnicotinamide, nifenazone, nicotinaldéhyde, acide isonicotinique, acide méthylisonicotinique, thionicotinamide, nialamide, acide 2-mercaptonicotinique, nicomol et niaprazine) ; la vitamine B5 (ou panthénol ou alcool panthénylique ou 2,4-dihydroxy-N-(3-hydroxypropyl)-3,3diméthylbutanamide), sous ses différentes formes : D-panthénol, DL-panthénol), et ses dérivés et analogues, tels que le panthoténate de calcium, la panthétine, la pantothéine, l'éther de éthyl panthényl, l'acide pangamique, la pyridoxine, le pantoyl lactose, et les composés naturels en contenant tels que la gelée royale ; la vitamine D et ses analogues tels que ceux décrits dans le document WO-A-00/26167 ; la vitamine F ou ses analogues tels que les mélanges d'acides insaturés possédant au moins une double liaison et notamment les mélanges d'acide linoléique, d'acide linolénique et d'acide arachidonique, ou les composés en contenant et notamment les huiles d'origine végétale en contenant, telles que par exemple l'huile de jojoba, et leurs mélanges.
4) Comme agents anti-élastase, on peut citer notamment les dérivés peptidiques, et notamment les peptides de graines de légumineuses tels que ceux commercialisés par les Laboratoires Sériobiologiques de Nancy sous la référence Parelastyl ; les dérivés de N-acylamino-amides décrits dans la demande FR-A-2,180,033, comme par exemple le {2-[acétyl-(3-trifluorométhyl-phényl)-amino]-3-méthyl-butyrylaminol acétate d'éthyle et l'acide {2-[acétyl-(3-trifluorométhylphényl)-amino]-3-méthyl-butyryl-amino} acétique, et leurs mélanges.
5) Comme agents anti-collagénase, on peut citer les inhibiteurs de métalloprotéase, tels que l'acide éthylènediamine (EDTA), la cystéine, et leurs mélanges.
6) Comme protides, on peut citer par exemple les protéines de blé ou de soja, leurs hydrolysats, comme ceux commercialisés par la société Silab sous la référence Tensine, et leurs mélanges.
7) Comme dérivés d'acide gras, on peut citer notamment les phospholipides polyinsaturés dont les phospholipides d'acide gras essentiels de poulpe, et leurs mélanges.
8) Comme stéroïdes, on peut citer par exemple la DHEA ou déhydroépiandrostérone, ses précurseurs biologiques, ses métabolites, et leurs mélanges. Par "précurseurs biologiques" de la DHEA, on entend notamment la Δ5-prégnénolone, la 17α-hydroxy prégnénolone et le sulfate de 17α-hydroxy prégnénolone. Par dérivés de la DHEA, on entend aussi bien ses dérivés métaboliques que ses dérivés chimiques. Comme dérivés métaboliques, on peut citer notamment le Δ5-androstène-3,17-diol et notamment le 5-androstène 3β, 17β-diol, la Δ4-androstène-3,17-dione, la 7 hydroxy DHEA (7α-hydroxy DHEA ou 7β-hydroxy-DHEA), la 7-céto-DHEA qui est elle-même un métabolite de la 7β-hydroxy DHEA et la benzoyl DHEA.
9) Comme oligo-éléments, on peut citer par exemple le cuivre, le zinc, le sélénium, le fer, le magnésium, le manganèse, et leurs mélanges.
10) Comme extraits d'algues, on peut citer les extraits d'algues rouges ou brunes, et par exemple l'extrait d'algues brunes de la famille des Laminaires, comme les extraits de l'espèce Laminaria digitata, et plus particulièrement celui vendu par la société CODIF sous la dénomination Phycosaccharides, qui est une solution concentrée d'un oligosaccharide comprenant l'enchaînement de deux acides uriques : acide mannuronique et l'acide guluronique.
11) Comme extraits de planctons, on peut citer le plancton en dispersion aqueuse (nom INCI : Vitreoscilla Ferment) commercialisé sous la dénomination MEXORYL SAH par la société Chimex.
12) Comme enzymes, on peut utiliser toute enzyme d'origine animale, microbiologique (bactérienne, fongique ou virale) ou synthétique (obtenue par synthèse chimique ou biotechnologique), sous forme cristalline pure ou sous une forme diluée dans un diluant inerte. On peut citer par exemple les lipases, les protéases, les phospholipases, les laccases, les cellulases, les peroxydases notamment les lactoperoxydases, les catalases, les superoxyde dismutases, ou parmi des extraits végétaux contenant les enzymes précitées, et leurs mélanges. Elles peuvent être choisies par exemple parmi celle vendue sous la dénomination commerciale « Subtilisine SP 554 » par la société Novo Nordisk et celle vendue sous la dénomination commerciale « LYSOVEG LS » par la société Laboratoires Sérobiologiques de Nancy.
13) Comme co-enzymes, on peut utiliser notamment l'ubiquinone ou coenzyme Q10 qui appartient à la famille des benzoquinones à chaîne alkylénée, le coenzyme R qui est la biotine (ou vitamine H), et leurs mélanges.
14) Comme flavonoïdes, on peut citer par exemple les isoflavonoïdes qui constituent une sous-classe des flavonoïdes, formés d'un squelette 3-phényl chromane qui peut comporter des substituants variés et différents niveaux d'oxydation. Le terme « isoflavonoïde » regroupe plusieurs classes de composés parmi lesquels on peut citer les isoflavones, les isoflavanones, les roténoïdes, les pterocarpans, les isoflavanes, les isoflavanes-3-enes, les 3-arylcoumarines, les 3-aryl-4-hydroxycoumarins, les coumestanes, les coumaronochromones, les méthyldeoxybenzoines, les 2-arylbenzofuranes, et leurs mélanges. Les isoflavonoïdes peuvent être d'origine naturelle ou synthétique. Par "origine naturelle", on entend l'isoflavonoïde à l'état pur ou en solution à différentes concentrations, obtenu par différents procédés d'extraction à partir d'un élément, généralement une plante, d'origine naturelle. Par "origine synthétique", on entend l'isoflavonoïde à l'état pur ou en solution à différentes concentrations, obtenu par synthèse chimique. Comme isoflavonoïdes d'origine naturelle, on peut citer la daidzine, la génistine, la daidzéine, la formononétine, la cunéatine, la génistéine, l'isoprunétine et la prunétine, la cajanine, l'orobol, la pratenséine, le santal, la junipégénine A, la glycitéine, l'afrormosine, la rétusine, la tectorigénine, l'irisolidone, la jamaicine, ainsi que leurs analogues et métabolites.
15) Comme céramides, on peut utiliser tout type de céramide, d'origine naturelle ou synthétique, par exemple de type II, de type III, de type IV, de type V ou de type VI, et leurs mélanges. On peut citer par exemple comme céramides, la N-oléoyldihydrosphingosine, la N-stéaroylphytosphingosine, le N-hydroxybéhénoyldihydrosphingosine, la N-hydroxypalmitoyldihydrosphingosine, la N-linoléoyldihydrosphingosine, la N-palmitoyldihydrosphingosine, la N-stéaroyldihydrosphingosine, la N-béhénoyldihydrosphingosine, et leurs mélanges.
16) Comme agents myorelaxants qui sont des agents de lissage des rides d'expression, on peut citer par exemple les sapogénines (voir document EP-A-1,352,643) ; l'adénosine et ses dérivés (voir document FR-0214828) ; les antagonistes des récepteurs associés aux canaux calciques (voir document FR-A-2,793,681), et en particulier le manganèse et ses sels (voir document FR-A-2,809,005) et l'alvérine (voir document FR-A-2,798,590) ; les agonistes des récepteurs associés aux canaux chlore, dont la glycine (voir document EP-A-0704210) et certains extraits d'*Iris pallida* (voir document FR-A-2,746,641).
17) Comme dérivés C-glycosides aptes à stimuler la synthèse des glycosaminoglycannes, on peut citer notamment les dérivés C-glycosides de formule suivante : 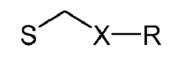 dans laquelle,
   - S représente un monosaccharide ou un polysaccharide jusqu'à 20 unités sucre, sous forme pyranose et/ou furanose et de série L et/ou D, ledit mono- ou polysaccharide présentant au moins une fonction hydroxyle obligatoirement libre et/ou éventuellement une ou plusieurs fonctions amine éventuellement protégée,
   - la liaison S-CH₂X représente une liaison de nature C-anomèrique,
   - X représente un groupement choisi parmi: -CO-, -CH(OH)-, -CH(NR₁R₂)-, -CHR'-, -C(=CHR')-,
   - R représente une chaîne alkyle, perfluoroalkyle, hydrofluoroalkyle linéaire ou ramifiée, saturée ou insaturée, un cycle cycloalkyle, cycloperfluoroalkyle, cyclohydrofluoroalkyle, comprenant de 1 à 18 atomes de carbone, un radical phényle ou benzyle, la dite chaîne, ledit cycle ou ledit radical pouvant être éventuellement interrompu par un ou plusieurs hétéroatomes choisi parmi l'oxygène, le souffre, l'azote, le silicium, et éventuellement substituée par au moins un radical choisi parmi -OR'₁, -SR"₁, -NR"'₁R'₂, -COOR"₂, -CONHR"'₂, -CN, halogène, perfluoroalkyle, hydrofluoroalkyle et/ou au moins un radical cycloalkyle, aryle, hétérocyclique éventuellement substitués, où
   - R', R₁, R₂, identiques ou différents ont la même définition que celle donnée pour R, et peuvent également représenter un hydrogène et un radical hydroxyle,
   - R'₁, R'₂, R"₁, R"₂, R"'₁, R"'₂, identiques ou différents, représentent un atome d'hydrogène, un radical choisi parmi un radical alkyle, hydroxyle, perfluoroalkyle et/ou hydrofluoroalkyle, linéaire ou ramifié, saturé ou insaturé, comprenant de 1 à 30 atomes de carbone,

Et notamment les composés décrits dans le document EP-A-1,345,919.

III) Les agents blanchissants ou dépigmentants, comme par exemple l'acide kojique et ses dérivés ; l'hydroquinone et ses dérivés tels que l'arbutine et ses esters ; la vitamine C et ses dérivés tels que l'ascorbyle phosphate de magnésium ; les sels tels que le calcium D pantéthéine sulfonate ; l'acide ellagique et ses dérivés ; le rucinol ; l'acide linoléique et ses dérivés ; les extraits de plantes, et notamment de réglisse, de mûrier ou de scutellaire ; le glutathion et ses précurseurs ; la cystéine et ses précurseurs ; les composés dérivés d'aminophénol décrits dans le document WO-A-99/10318, comme notamment le N-éthyloxycarbonyl-4-amino-phénol, le N-éthyloxycarbonyl-O-éthyloxycarbonyl-4-amino-phénol, le N-cholestéryloxycarbonyl-4-aminophénol, le N-éthylaminocarbonyl-4-aminophénol ; et les mélanges de ces composés.

IV) Les agents de coloration de la peau, tels que la Dihydroxy acétone (DHA), les colorants naturels tels que les extraits végétaux comme par exemple les extraits de sorgho, mais aussi les azurants optiques tels que le di-styryl-4,4'bi-phenyl disulfonate commercialisés par la société Ciba Geigy sous le nom de Tinopal CBS-X ®. On peut aussi citer par exemple le 4,4'-bis[(4,6-diamilino-1,3,5-triazin-2-yl)amino]stilbène-2,2'-disulfonate de sodium, et le 2,5 thiophène di-yl bis(5 terbutyl-1,3 benzoxazole) commercialisés par la société Ciba Geigy sous les noms de Tinopal SOP ® et d'Uvitex OB ®.

V) Les filtres solaires, qui peuvent être choisis parmi les filtres chimiques UVA et UVB ou les filtres physiques habituellement utilisables dans le domaine cosmétique.

Comme filtres UVB, on peut citer par exemple :
(1) les dérivés de l'acide salicylique, en particulier le salicylate d'homomenthyle et le salicylate d'octyle ;
(2) les dérivés de l'acide cinnamique, en particulier le p-méthoxycinnamate de 2-éthylhexyle, commercialisé par la société Givaudan sous la dénomination Parsol MCX;
(3) les dérivés de β,β'-diphénylacrylate liquides, en particulier l'α-cyano-α,β' diphénylacrylate de 2-éthylhexyle ou octocrylène, commercialisé par la société BASF sous la dénomination UVINUL N539 ;
(4) les dérivés de l'acide p-aminobenzoïque ;
(5) le 4-méthyl benzylidène camphre commercialisé par la société Merck sous la dénomination EUSOLEX 6300 ;
(6) l'acide 2-phénylbenzimidazole 5-sulfonique commercialisé sous la dénomination EUSOLEX 232 par la société Merck ;
(7) les dérivés de 1,3,5-triazine, en particulier :
   - la 2,4,6-tris[p-(2'-éthylhexyl-1'-oxycarbonyl)anilino]-1,3,5-triazine commercialisé par la société BASF sous la dénomination UVINUL T150, et
   - le dioctyl butamido triazone commercialisé par la société Sigma 3V sous la dénomination UVASORB HEB ;
(8) les mélanges de ces filtres.

Comme filtres UVA, on peut citer par exemple :
(1) les dérivés de dibenzoylméthane, en particulier le 4-(tert.-butyl) 4'-méthoxy dibenzoylméthane commercialisé par la société Givaudan sous la dénomination PARSOL 1789 ;
(2) l'acide benzène 1,4 [di(3-méthylidènecampho-10-sulfonique)] éventuellement sous forme partiellement ou totalement neutralisée, commercialisé sous la dénomination MEXORYL SX par la société Chimex.
(3) les dérivés de benzophénone, par exemple :
   - la 2,4-dihydroxybenzophénone (benzophénone-1) ;
   - la 2,2',4,4'-tétra-hydroxybenzophénone (benzophénone-2) ;
   - la 2-hydroxy-4-méthoxy-benzophénone (benzophénone-3), commercialisé sous la dénomination UVINUL M40 par la société BASF ;
   - l'acide 2-hydroxy-4-méthoxy-benzophénone-5-sulfonique (benzophénone-4) ainsi que sa forme sulfonate (benzophénone-5), commercialisé par la société BASF sous la dénomination UVINUL MS40 ;
   - la 2,2'-dihydroxy-4,4'-diméthoxy-benzophénone (benzophénone-6) ;
   - la 5-chloro-2-hydroxybenzophénone (benzophénone-7) ;
   - la 2,2'-dihydroxy-4-méthoxy-benzophénone (benzophénone-8) ;
   - le sel disodique du diacide 2,2'-dihydroxy-4,4'-diméthoxy-benzophénone-5,5'-disulfonique (benzophénone-9) ;
   - la 2-hydroxy-4-méthoxy-4'-méthyl-benzophénone (benzophénone-10) ;
   - la benzophénone-11 ;
   - la 2-hydroxy-4-(octyloxy)benzophénone (benzophénone-12).
(4) les dérivés silanes ou les polyorganosiloxanes à groupement benzophénone ;
(5) les anthranilates, en particulier l'anthranilate de menthyle commercialisé par la société Haarman & Reiner sous la dénomination NEO HELIOPAN MA ;
(6) les composés comportant par molécule au moins deux groupes benzoazolyle
   ou au moins un groupe benzodiazolyle, en particulier l'acide 1,4-bis-benzimidazolyl-phenylèn-3,3',5,5'-tétrasulfonique ainsi que ses sels commercialisés par la société Haarman & Reimer ;
(7) les dérivés siliciés de benzimidazolyl-benzazoles N-substitués ou de benzofuranyl-benzazoles, et en particulier :
   - le 2-[1-[3-[1,3,3,3-tétraméthyl-1-[(triméthylsilyl)oxy]disiloxanyl]propyl]-1H-benzimidazol-2-yl]-benzoxazole ;
   - le 2-[1-[3-[1,3,3,3-tétraméthyl-1-[(triméthylsilyl)oxy]disiloxanyl]propyl]-1H-benzimidazol-2-yl]-benzothiazole ;
   - le 2-[1-(3-trimethylsilanyl-propyl)-1H-benzimidazol-2-yl]-benzoxazole ;
   - le 6-méthoxy-1,1'-bis-(3-triméthylsilanyl-propyl)-1H,1'H-[2,2']bibenzimidazolyl-benzoxazole ;
   - le 2-[1-(3-trimethylsilanyl-propyl)-1 H-benzimidazol-2-yl]-benzothiazole, qui sont décrits dans le document EP-A-1 028 120 ;
(8) les dérivés de triazine, et en particulier la 2,4-bis {[4-(2-éthyl-hexyloxy)-2-hydroxy]-phényl}-6-(4-méthoxy-phényl)-1,3,5-triazine commercialisé par la société Ciba Geigy sous la dénomination TINOSORB S, et le 2,2'-méthylènebis-[6-(2H benzotriazol-2-yl)4-(1,1,3,3-tétraméthylbutyl)phénol] commercialisé par la société Ciba Geigy sous la dénomination TINOSORB M ;
(9) Les silicones benzotriazoles, qui sont décrits notamment dans le document EP-A- 0392 883, en particulier le silicone benzotriazole de formule : 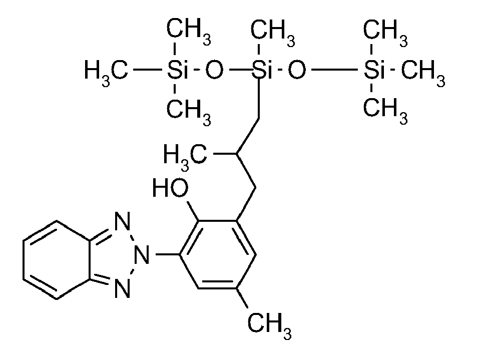
(10) leurs mélanges.

On peut aussi utiliser un mélange de plusieurs de ces filtres.

Comme filtres physiques, on peut citer les oxydes de titane (dioxyde de titane amorphe ou cristallisé sous forme rutile et/ou anatase), de zinc, de fer, de zirconium, de cérium ou leurs mélanges. Ces oxydes métalliques peuvent être sous forme de particules ayant une taille micrométrique ou nanométrique (nano-pigments). Sous forme de nano-pigments, les tailles moyennes des particules vont par exemple de 5 à 100 nm. On utilise de préférence des nano-pigments.

VI) Les anti-cernes tels que la Vitamine K1 et ses dérivés.

VII) Les actifs antimicrobiens, antifongiques ou anti-acnéiques, notamment les hydroxyacides tels que l'acide salicylique et ses dérivés, l'acide glycolique, l'acide lactique l'acide ellagique, le 2,4,4'-trichloro-2'-hydroxy diphényl éther (ou triclosan), le 3,4,4'-trichlorobanilide (ou triclocarban), le phénoxyéthanol, le phénoxypropanol, le phénoxyisopropanol, l'hexamidine iséthionate, le métronidazole et ses sels, le miconazole et ses sels, l'itraconazole, le terconazole, l'éconazole, le ketoconazole, le saperconazole, le fluconazole, le clotrimazole, le butoconazole, l'oxiconazole, le sulfaconazole, le sulconazole, le terbinafine, le ciclopiroxe, le ciclopiroxolamine, l'acide undécylenique et ses sels, le peroxyde de benzoyle, l'acide 3-hydroxy benzoïque, l'acide 4-hydroxy benzoïque, l'acide phytique, l'acide N-acétyl-L-cystéine, l'acide lipoïque, l'acide azélaïque et ses sels, l'acide arachidonique, le résorcinol, le 2,4,4'-trichloro-2'-hydroxydiphényl éther, le 3,4,4'-trichlorocarbanalide, l'octopirox, l'octoxyglycérine, l'octanoylglycine, le caprylyl glycol, l'acide 10-hydroxy-2-décanoïque, le dichlorophenyl imidazol dioxolan et ses dérivés décrits dans le document WO-A-93/18743, le farnesol, les phytosphingosines, les dérivés du sélénium, le pyrithione zinc, les tétracyclines comme l'érythromycine, et les mélanges de ces composés.

VIIII) les agent anti-séborrhéiques, choisis par exemple parmi :
- le soufre et les dérivés soufrés ;
- les sels de zinc tels que le lactate, le gluconate, le pidolate, le carboxylate, le salicylate et/ou le cystéate de zinc ;
- le chlorure de sélénium ;
- la vitamine B6 ou pyridoxine ;
- le mélange de capryloyl glycine, de sarcosine et d'extrait de cinnamomum zeylanicum commercialisé notamment par la société SEPPIC sous la dénomination commerciale Sepicontrol A5^{®} ;
- un extrait de Laminaria saccharina commercialisé notamment par la société SECMA sous la dénomination commerciale Phlorogine^{®} ;
- un extrait de Spiraea ulmaria commercialisé notamment par la société SILAB sous la dénomination commerciale Sebonormine^{®} ;
- des extraits de végétaux des espèces Arnica montana, Cinchona succirubra, Eugenia caryophyllata, Humulus lupulus, Hypericum perforatum, Mentha piperita, Rosmarinus officinalis, Salvia oficinalis et Thymus vulgaris, tous commercialisés par exemple par la société MARUZEN ;
- un extrait de Serenoa repens commercialisé notamment par la société EUROMED;
- des extraits de plantes du genre Silybum ;
- des extraits végétaux contenant des sapogénines et en particulier les extraits de Dioscorées riches en diosgénine ou hécogénine ;
- des extraits d'Eugenia caryophyllata contenant de l'eugénol et du glucoside d'eugényle ;
et leurs mélanges.

IX) Les agents anti transpirant et déodorants :
Comme agents antitranspirants, on peut citer par exemple les sels d'aluminium et/ou de zirconium, comme par exemple le chlorhydrate d'aluminium, l'aluminium chlorohydrex, l'aluminium chlorohydrex PEG, l'aluminium chlorohydrex PG, l'aluminium dichlorohydrate, l'aluminium dichlorohydrex PEG, l'aluminium dichlorohydrex PG, l'aluminium sesquichlorohydrate, l'aluminium sesquichlorohydrex PEG, l'aluminium sesquichlorohydrex PG, les sels d'alun, l'aluminium sulfate, l'aluminium zirconium octachlorohydrate, l'aluminium zirconium pentachlorohydrate, l'aluminium zirconium tétrachlorohydrate, l'aluminium zirconium trichlorohydrate et plus particulièrement l'aluminium chlorhydrate commercialisé par la société REHEIS sous la dénomination REACH 301 ou 303 ou par la société GUILINI CHEMIE sous la dénomination ALOXICOLL PF 40, et le sel d'aluminium et de zirconium commercialisé par la société REHEIS sous la dénomination REACH AZP-908-SUF ; les complexes d'hydroxychlorure de zirconium et d'hydroxychlorure d'aluminium avec un acide aminé tels que ceux décrits dans le document US-A-3,792,068, communément connus sous l'appellation "ZAG complexes", comme par exemple l'aluminium zirconium octachlorohydrex GLY, l'aluminium zirconium pentachlorohydrex GLY, l'aluminium zirconium tetrachlorohydrate GLY et l'aluminium zirconium trichlorohydrate-GLY.
Comme agents déodorants, on peut citer par exemple le pyrrolidone carboxylate de zinc (plus communément appelé pidolate de zinc), le sulfate de zinc, le chlorure de zinc, le lactate de zinc, le gluconate de zinc et le phénolsulfonate de zinc, le 2,4,4'-trichloro-2'-hydroxydiphényléther (Triclosan), le 2,4-dichloro-2'-hydroxydiphényléther, le 3',4',5'-trichlorosalicylanilide, le 1-(3',4'-dichlorophenyl)-3-(4'-chlorophenyl)urée (Triclocarban) ou le 3,7,11-triméthyldodéca-2,5,10-triénol (Farnesol) ; les sels d'ammonium quaternaires comme les sels de cetyltrimethylammonium, les sels de cétylpyridinium ; la chlorhexidine et les sels; le monocaprate de diglycérol, le monolaurate de diglycérol, le monolaurate de glycérol ; les sels de polyhexaméthylène biguanide.

X) Les actifs apaisants tels que les triterpènes pentacycliques et les extraits de plantes (ex : Glycyrrhiza glabra) en contenant comme l'acide β-glycyrrhétinique et ses sels et/ou ses dérivés (l'acide glycyrrhétinique monoglucuronide, le stéaryl glycyrrhétinate, l'acide 3- stéaroyloxy glycyrrhétique) ; l'acide ursolique et ses sels ; l'acide oléanolique et ses sels ; l'acide bétulinique et ses sels ; les extraits de Paeonia suffruticosa, de lactiflora, de Laminaria saccharina, de camomille, du Pygeum, de Boswellia serrata, de Centipeda cunnighami, d'Helianthus annuus, de Linum usitatissimum, de Cola nitida, de clou de girofle, d'Epilobium Angustifolium, de Bacopa monieri ; les sels de l'acide salicylique et en particulier le salicylate de zinc ; les phycosaccharides de la société Codif ; l'huile de Canola ; le bisabolol ; l'allantoïne ; le Sépivital EPC (diesterphosphorique de vitamine E et C) de la société Seppic ; les huiles insaturées en oméga 3 telles que les huiles de rosier muscat, de cassis, d'Echium, de poisson ; la capryloyl glycine ; le Seppicalm VG (sodium palmitoylproline et nymphea alba) de la société Seppic ; les tocotrienols ; le piperonal , l'aloe vera ; les phytostérols.

XI) Les actifs lipolytiques ou amincissants, c'est-à-dire ayant une activité favorable, directe ou indirecte, sur la diminution du tissu adipeux, tels que :
- les dérivés xanthiques comme la caféine et ses dérivés, notamment les 1-hydroxyalkylxanthines décrites dans le document FR-A-2,617,401, la caféine citrate, la théophylline et ses dérivés, la théobromine, l'acéfylline, l'aminophylline, le chloroéthylthéophylline, le diprofylline, le diniprophylline, l'étamiphylline et ses dérivés, l'étofylline, la proxyphylline ; ou les associations contenant des dérivés xanthiques, comme l'association de caféine et de silanol (dérivé méthylsilanetriol de caféine), et par exemple le produit commercialisé par la société Exsymol sous la dénomination caféisilane C ;ou bien les composés d'origine naturelle contenant des bases xanthiques et notamment de la caféine, tels que les extraits de thé, de café, de guarana, de maté, de cola (*Cola Nitida*) et notamment l'extrait sec de fruit de guarana (*Paulina sorbilis*) contenant 8 à 10 % de caféine ; l'éphédrine et ses dérivés qui peuvent notamment se retrouver à l'état naturel dans les plantes telles que le Ma Huang (Ephedra plant) ;
- les extraits végétaux et les extraits d'origine marine, qui soit sont actifs sur les récepteurs à inhiber, tels que les β-2-bloqueurs, les NPY-bloqueurs (décrits dans le document EP-A-838217), soit inhibent la synthèse des récepteurs aux LDL ou VLDL, soit sont actifs pour stimuler les récepteurs β et les protéines G, conduisant à l'activation de l'adénylcyclase. Comme extraits végétaux de ce type, on peut citer par exemple :
   - le *Garcinia Cambogia,*
   - les extraits de *Bupleurum chinensis,*
   - les extraits de lierre grimpant (*Hedera Helix*), d'arnica (*Arnica Montana L*), de romarin (*Rosmarinus officinalis N*), de souci (*Calendula officinalis*), de sauge (*Salvia officinalis L*), de ginseng (*Panax ginseng*), de millepertuis (*Byperycum Perforatum*), de fragon (*Ruscus aculeatus L*), d'ulmaire (*Filipendula ulmaria L*), d'orthosiphon (*Orthosiphon Stamincus Benth*), de bouleau (*Betula alba*), de cécropia et d'arganier,
   - les extraits de ginkgo biloba,
   - les extraits de prêle,
   - les extraits d'escine,
   - les extraits de cangzhu,
   - les extraits de *chrysanthellum indicum,*
   - les extraits de dioscorés riches en diosgénine ou la diosgénine ou hécogénine pure et leurs dérivés.
   - les extraits des plantes du genre *Armeniacea, Atractylodis Platicodon, Sinom-menum, Pharbitidis, Flemingia,*
   - les extraits de *Coleus* tels que *C. Forskohlii*, *C. blumei*, *C. esquirolii*, *C. scutellaroïdes*, *C. xanthantus* et *C. Barbatus,* tel que l'extrait de racine de *Coleus Barbatus* contenant 60 % de forskoline,
   - les extraits de Ballote,
   - les extraits de *Guioa,* de *Davallia*, de *Terminalia,* de *Barringtonia,* de *Trema,* d'*Antirobia.*
      Comme extraits d'origine marine on peut citer :
   - les extraits d'algues ou de phytopancton, tels que le rhodystérol ou l'extrait de *Laminaria Digitata* commercialisé sous la dénomination PHYCOX75 par la société Secma, l'algue skeletonema décrite dans le document FR-A-2,782,921 ou les diatomées décrites dans le document FR-A-2,774,292.

XII) Les agents tenseurs.
Comme agents tenseurs, on peut citer par exemple :
- les polymères synthétiques ;
- les polymères d'origine naturelle ;
- les silicates mixtes ;
- les particules colloïdales de charges inorganiques.

- Les polymères synthétiques utilisables en tant qu'agent tenseur peuvent être choisis parmi :
   - les polymères et copolymères de polyuréthanne ;
   - les polymères et copolymères acryliques ;
   - les polymères siliconés greffés ;
   - les polymères hydrosolubles ou hydrodispersibles comprenant des unités hydrosolubles ou hydrodispersibles et des unités à LCST (Lower Critical Solution
   Temperature) comme les polymères décrits dans le document FR-A-2,819,429.
- Les polymères d'origine naturelle utilisables en tant qu'agent tenseur peuvent être choisis parmi :
   - les protéines végétales et hydrolysats de protéines végétales ;
   - les polysaccharides d'origine végétale, éventuellement sous forme de microgels, tels que l'amidon ;
   - les latex d'origine végétale
   Les silicates mixtes peuvent être d'origine naturelle ou synthétique, et ils peuvent renfermer plusieurs types de cations choisis parmi les métaux alcalins (par exemple Na, Li, K) ou alcalino-terreux (par exemple Be, Mg, Ca) et les métaux de transition. On utilise de préférence des phyllosilicates, à savoir des silicates ayant une structure dans laquelle les tétraèdres SiO4 sont organisés en feuillets entre lesquels se trouvent enfermés les cations métalliques. Une famille de silicates particulièrement préférée comme agents tenseurs est celle des laponites. Les laponites sont des silicates de magnésium, de lithium et de sodium ayant une structure en couches semblable à celle des montmorillonites. La laponite est la forme synthétique du minéral naturel appelé "hectorite". On peut utiliser par exemple la laponite commercialisée sous la dénomination Laponite XLS ou Laponite XLG par la société Rockwood.
- Une autre classe encore d'agents tenseurs est constituée par les microparticules de cire. Il s'agit de particules ayant un diamètre généralement inférieur à 5 µm, ou mieux à 0,5 µm, et constituées essentiellement d'une cire ou d'un mélange de cires choisies par exemple parmi les cires de Carnauba, de Candelilla ou d'Alfa. Le point de fusion de la cire ou du mélange de cires est de préférence compris entre 50°C et 150°C.
- En variante encore, on peut utiliser comme agent tenseur, des particules colloïdales de charges inorganiques. Par "particules colloïdales", on entend des particules colloïdales en dispersion dans un milieu aqueux, hydroalcoolique ou alcoolique, ayant un diamètre moyen en nombre compris entre 0,1 et 100 nm, de préférence entre 3 et 30 nm. Comme exemples de charges inorganiques, on peut citer la silice, l'oxyde de cérium, l'oxyde de zirconium, l'alumine, le carbonate de calcium, le sulfate de baryum, le sulfate de calcium, l'oxyde de zinc et le dioxyde de titane. Une charge inorganique particulièrement préférée est la silice. Des particules colloïdales de silice sont notamment disponibles sous forme de dispersion aqueuse de silice colloïdale auprès de la société catalysts & Chemicals sous les dénominations commerciales COSMO S-40 et COSMO S-50. on peut aussi utiliser des particules colloïdales composites silice-alumine, telles que celles commercialisées par la société Grace sous les noms de Ludox AM, Ludox HSA et Ludox TMA.

XIII) Les charges matifiantes, le terme «charge matifiante» désignant une particule sphérique ou non sphérique, poreuse ou non poreuse, présentant un indice de réfraction inférieur ou égal à 2,2, notamment inférieur ou égal à 2, et en particulier inférieur ou égal à 1,8, de préférence allant de 1,3 à 1,6. Les charges matifiantes selon l'invention ont généralement une taille en volume inférieure à 15 µm.

Dans un mode préférentiel de l'invention les charges matifiantes sont sphériques.

Dans un mode préférentiel de l'invention les charges matifiantes sont poreuses. Dans ce cas, la surface spécifique des particules que l'on peut relier à la porosité est supérieure à 10 m²/g, de préférence supérieure à 50 m²/g.

Plus particulièrement, ces charges peuvent par exemple être choisies parmi :
- les microparticules poreuses de silice, comme par exemple les Silica beads SB150 et SB700 de la société Miyoshi, ayant une taille moyenne 5 microns; les Sunspheres Série-H de la société Asahi Glass, comme par exemple les Sunsphère H33, H51, et H53 de taille respectives 3, 5 et 5 microns ;
- les poudres de polytétrafluoroéthylène, comme les PTFE Ceridust 9205F de la société Clariant, ayant une taille moyenne 8 microns;
- les poudres de résine de silicone comme les Silicon resin Tospearl 145A de la société GE Silicone, ayant une taille moyenne 4,5 microns;
- les particules hémisphériques creuses de silicone comme les NLK 500, NLK 506 et NLK 510 de la société Takemoto Oil and Fat.
- les poudres de copolymères acryliques, notamment de poly(méth)acrylate de méthyle, comme les particules PMMA Jurymer MBI de la société Nihon Junyoki, ayant une taille moyenne 8 microns, les sphères creuses de PMMA vendues sous la dénomination Covabead LH85 par la société Wackherr, les microsphères de polyméthacrylate de méthyle, commercialisées sous la dénomination MICROSPHERE M-100 par la société Matsumoto; ou les microsphères commercialisées sous la dénomination MICROPEARL F 80 ED par la société Matsumoto ;
- les poudres de cire, comme les particules « Paraffin wax microease 114S » de la société Micropowders, ayant une taille moyenne 7 microns;
- les poudres de polyéthylène, notamment comprenant au moins un copolymère éthylène/acide acrylique, et en particulier constituées de copolymères éthylène/acide acrylique comme les particules Flobeads EA 209 de la société Sumitomo (de taille moyenne 10 microns;
- les poudres d'organopolysiloxane élastomèrique réticulé, enrobées de résine de silicone, notamment de résine silsesquioxane, comme décrit par exemple dans le document US-A-5,538,793. De telles poudres d'élastomère sont vendues sous les dénominations « KSP-100 », « KSP-101 », « KSP-102 », « KSP-103 », « KSP-104 », « KSP-105 » par la société SHIN ETSU ;
- les poudres composites de talc/dioxyde de titane/alumine/silice comme celles vendues sous la dénomination Coverleaf AR-80 par la société Catalyst & chemicals ;
- les poudres de polyamide (Nylon®), comme par exemple les particules de Nylon 12 du type Orgasol de la société Atofina, ayant une taille moyenne 10 microns;
- les microsphères à base de copolymères acryliques, telles que celles en copolymère diméthacrylate d'éthylène glycol/ methacrylate de lauryle vendues par la société Dow Corning sous la dénomination de POLYTRAP ;
- les poudres expansées telles que les microsphères creuses et notamment, les microsphères formées d'un terpolymère de chlorure de vinylidène, d'acrylonitrile et de méthacrylate et commercialisées sous la dénomination EXPANCEL par la société Kemanord Plast sous les références 551 DE 12 (granulométrie d'environ 12 µm et masse volumique 40 kg/m3), 551 DE 20 (granulométrie d'environ 30 µm et masse volumique 65 kg/m3), 551 DE 50 (granulométrie d'environ 40 µm),
- les poudres de copolymère éthylène-acrylate, comme celles commercialisées sous la dénomination FLOBEADS par la société Sumitomo Seika Chemicals ;
- les poudres de matériaux organiques naturels, telles que les poudres d'amidon, notamment d'amidons de maïs, de blé ou de riz, réticulés ou non, comme les poudres d'amidon réticulé par l'anhydride octényl-succinate, commercialisées sous la dénomination DRY-FLO par la société National Starch ;
- les microbilles de cellulose et les fibres.
- et leurs mélanges.

La composition selon l'invention peut en outre renfermer diverses charges additionnelles d'origine minérale ou organique. Elles peuvent être de toute forme, notamment plaquettaires, sphériques ou oblongues, quelle que soit leur forme cristallographique (par exemple feuillet, cubique, hexagonale, orthorhombique, etc...).

Parmi les charges additionnelles utilisables dans la composition selon l'invention, on peut notamment citer le talc, le mica, le kaolin, de poly-β-alanine et de polyéthylène, la lauroyl-lysine, l'amidon, le nitrure de bore, le carbonate de calcium précipité, le carbonate et l'hydrocarbonate de magnésium, le sulfate de baryum, l'hydroxyapatite, les microcapsules de verre ou de céramique et les savons métalliques dérivés d'acides organiques carboxyliques ayant de 8 à 22 atomes de carbone, notamment de 12 à 18 atomes de carbone, par exemple le stéarate de zinc, de magnésium ou de lithium, le laurate de zinc, le myristate de magnésium.

XIV) Les actifs des traitements capillaires, tels que (1) les agents inhibiteurs de la chute du cheveu ainsi que les agents stimulateurs de la pousse du cheveu, tels que le minoxidil, la biotine, l'aminexil, la cystéine, le finastèride, le 2,4 dipirymidine N-oxyde, le panthénol et dérivés, la flavanone T, les antagonistes de calcium comme le diltiazem, le vérapamil, l'alvérine, et la nifédipine, les hormones comme la progestérone, les agonistes du récepteur FP comme le latanoprost, les inhibiteurs de la 15-hydroxy prostaglandine déshydrogénase de type 1, les prostaglandines et leurs dérivés, ou plus généralement tout extrait végétal possédant une activité anti-5-alpha réductase de type I ou II ; (2) les agents antipelliculaires tels que la pyrithione de zinc, les dérivés de 1-hydroxy-2-pyrrolidone ou encore les sulfures de sélénium.

La composition aqueuse et/ou le film peut contenir aussi les adjuvants habituellement utilisés dans le domaine cosmétique ou dermatologique, tels que colorants, pigments, parfums et huiles essentielles, des électrolytes, des actifs cosmétiques, des ajusteurs de pH (acide ou base), les conservateurs.

La composition aqueuse peut contenir aussi des polymères qui peuvent être utiles pour apporter des propriétés rhéologiques particulières à la composition aqueuse. Comme polymères, on peut citer par exemple les polymères carboxyvinyliques modifiés ou non, tels que les produits commercialisés sous les dénominations Carbopol (nom INCI : carbomer) et Pemulen (nom INCI : Acrylates/C10-30 alkyl acrylate crosspolymer) par la société Noveon ; les polyacrylates et polyméthacrylates tels que les produits vendus sous les dénominations de Lubrajel et Norgel par la société GUARDIAN ou sous la dénomination Hispagel par la société HISPANO CHIMICA ; les polyacrylamides ; les polymères et copolymères d'acide 2-acrylamido-2-méthylpropane-sulfonique, éventuellement réticulés et/ou neutralisés, comme le poly(acide 2-acrylamido 2-méthylpropane sulfonique) commercialisé par la société CLARIANT sous la dénomination « Hostacerin AMPS » (nom INCI : ammonium polyacryldimethyltauramide) ; les copolymères anioniques réticulés d'acrylamide et d'AMPS, se présentant sous la forme d'une émulsion E/H, tels ceux commercialisés sous le nom de SEPIGEL 305 (nom INCI : Polyacrylamide / C13-14 Isoparaffin / Laureth-7) et sous le nom de SIMULGEL 600 (nom INCI : Acrylamide / Sodium acryloyldimethyltaurate copolymer / Isohexadecane / Polysorbate 80) par la société SEPPIC ; les biopolymères polysaccharidiques comme la gomme de xanthane, la gomme de guar, la gomme de caroube, la gomme d'acacia, les scléroglucanes, les dérivés de chitine et de chitosane, les carraghénanes, les gellanes, les alginates, les celluloses telles que la cellulose microcristalline, la carboxyméthylcellulose, l'hydroxyméthylcellullose et l'hydroxypropylcellulose ; les copolymères d'anhydride maléique tels que le copolymère methyl vinyl ether / anhydride maleique réticulé par 1,9-decadiene (nom INCI : PVM/MA Decadiene Crosspolymer) commercialisé sous la dénomination Stabileze OM ou 06 par la société ISO ; les polymères associatifs comme les polyuréthannes associatifs, copolymères comportant au moins deux chaînes lipophiles hydrocarbonées ayant de 6 à 30 atomes de carbone, séparées par une séquence hydrophile, tels que les polyuréthannes commercialisés sous les dénominations SERAD FX1010, SERAD FX1100 et le SERAD FX1035 par la société HÜLS (nom INCI: Polyurethane), ceux commercialisés sous les dénominations Rheolate 255, Rheolate 278 et Rheolate 244 par la société RHEOX (nom INCI : Polyether-urea-polyurethane), ceux commercialisés sous les dénominations DW 1206F, DW 1206J, DW 1206B, DW 1206G par la société Röhm & Haas (nom INCI : Polyurethane), et celui commercialisé sous la dénomination Acrysol RM 2020 de la société Röhm & Haas. On peut aussi utiliser des polymères fixant tels que ceux classiquement utilisés dans les laques et les produits coiffants des cheveux.

Les adjuvants cités ci-dessus peuvent être présents dans le produit final dans les quantités habituelles dans le domaine considéré, ces quantités dépendant du composé utilisé et du but recherché. Les adjuvants peuvent être présents par exemple en une quantité allant de 0,001 à 20 % en poids, et mieux de 0,01 à 10 % en poids par rapport au poids de produit final.

Le kit selon l'invention peut être utilisé notamment pour obtenir des produits destinés à être appliqués sur la peau, les muqueuses, les phanères ou les cheveux, notamment comme produits de soin de la peau ou comme produits de maquillage de la peau ou comme produits capillaires ou comme produits solaires.

Les exemples ci-après de compositions selon l'invention sont donnés à titre d'illustration et sans caractère limitatif. Les quantités y sont données en % en poids, sauf mention contraire, et les noms sont indiqués en nom chimique ou en nom INCI selon les cas.

### Exemple 1 et exemple comparatif : film anhydre

| | Exemple comparatif | Exemple 1 selon l'invention |
|---|---|---|
| composition | Quantité en % | Quantité en % |
| Alcool polyvinylique (Celvol 540 PVA) | 2,1 | 2,0 |
| Hydroxypropyl cellulose (Klucel EF) | 3,2 | 3,1 |
| Eau | 87,0 | 85,3 |
| Carraghénane | 1,0 | 1,0 |
| Amidon de maïs estérifié (Dry Flo Plus) | 1,6 | 1,6 |
| Glycérine | 3,2 | 3,1 |
| Polyéthylène glycol 400 | 1,9 | 1,9 |
| PEG-12 Dimethicone (SILSOFT 880) | - | 2,0 |

### Mode opératoire de préparation du film :

Le mode opératoire a compris deux étapes. Dans un premier temps, on a réalisé le mélange des ingrédients, et dans un second temps, on a étalé ce mélange puis on l'a séché pour former le film.

### 1) Préparation de la solution

Les constituants indiqués sur le tableau ci-dessus ont été mélangés en deux étapes comme décrit ci-dessous, puis le mélange a été homogénéisé à l'aide d'un mélangeur à turbine (Rayneri).

Le mode opératoire de la préparation de la solution a été fait en deux étapes de la manière suivante :
1ère étape : on a dispersé les polymères (alcool polyvinylique et hydroxypropyl cellulose) dans l'eau et on a agité à vitesse et temps nécessaires pour obtenir un gel homogène. Cette étape a duré 3 heures avec une agitation forte (1200 à 1600 tpm).
2ème étape : on a dispersé le carraghénane et, pour l'exemple selon l'invention, le polysiloxane oxyéthyléné, et on a mélangé jusqu'à obtention d'un mélange parfaitement homogène. Une fois le mélange homogénéisé, on a ajouté l'amidon, le PEG 400 et la glycérine en agitant à la même vitesse que précédemment. Cette étape a duré 20 minutes.

### 2) Formation du film

Le couchage du mélange a été fait à l'aide d'une étireuse de film sur une feuille de SILPHAN S100 M44A sur la face non siliconée. L'appareil utilisé était un applicateur automatique de film de Braive Instruments. L'épaisseur humide choisie était de 700 µm. Les films ont ensuite été placés dans une étuve à 55°C sans contrôle d'humidité pendant 24 heures.

Après séchage, on a obtenu des films anhydres de 50 µm d'épaisseur environ, dont la composition était la suivante :

| | Exemple comparatif | Exemple 1 selon l'invention |
|---|---|---|
| composition | Quantité en % | Quantité en % |
| Alcool polyvinylique (Celvol 540 PVA) | 16,2 | 13,6 |
| Hydroxypropyl cellulose (Klucel EF) | 24,6 | 21,1 |
| Carraghénane | 7,7 | 6,8 |
| Amidon de maïs estérifié (Dry Flo Plus) | 12,3 | 10,9 |
| Glycérine | 24,6 | 21,1 |
| Polyéthylène glycol 400 | 14,6 | 12,9 |
| PEG-12 Dimethicone (SILSOFT 880) | - | 13,6 |

### Test

On a effectué un test de dissolution pour déterminer les propriétés de dissolution du film de l'exemple 1 comparativement à celui de l'exemple comparatif.

### Description de la méthode :

La méthode utilisée consiste à mesurer le temps qu'il faut à une formulation fluide (appelée ci-dessous jus) pour traverser un film hydrosoluble, en utilisant pour ce faire, une cellule de Franz détournée de son utilisation habituelle.

Le film a été placé à l'aide d'une pince entre la partie supérieure et la partie inférieure d'une cellule de Franz. Une quantité connue du jus a été déposée sur le film à l'aide d'une pipette Finn-pipette (20-200µL) ou une pipette Microman (0-50µL), sans qu'il n'y ait contact entre la pointe de la pipette et le film.

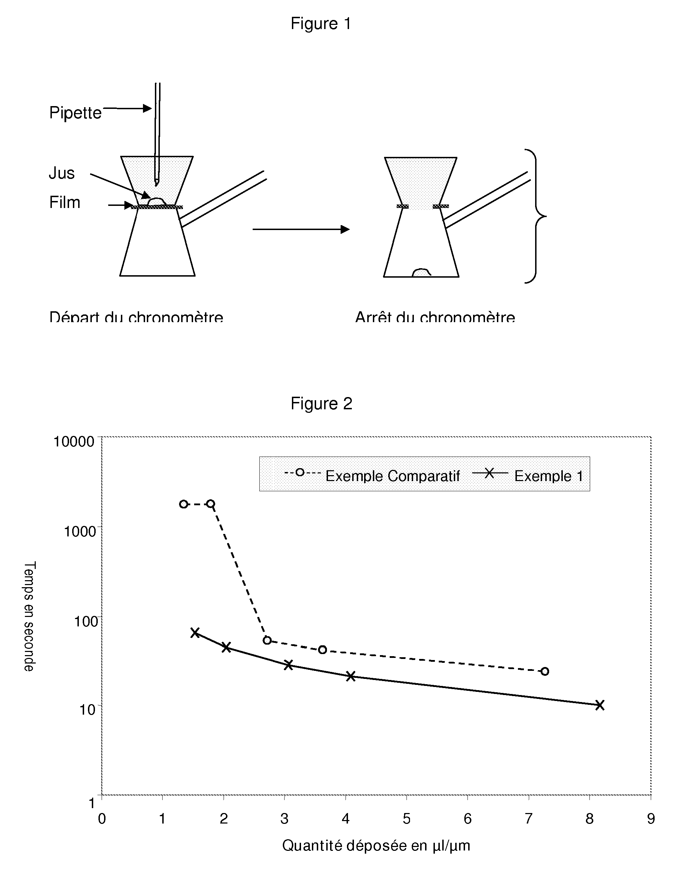

Le dispositif du test est représenté sur la figure 1.

Le chronomètre a été déclenché lorsque la goutte de jus a été déposée sur le film. Le chronomètre a été ensuite arrêté lorsque la goutte déposée a traversé le film et a touché le fond de la cellule de Franz.

Il a été mesuré ainsi le temps nécessaire à des quantités de jus variant de 50 à 400 µl pour traverser le film. On a ensuite utilisé ces valeurs pour construire une courbe représentant le temps pour traverser le film (en seconde) en fonction de la quantité de jus déposé, normalisée par l'épaisseur du film soit 50 µm (en µL/µm).

Les courbes obtenues avec les films correspondant aux exemples selon l'invention (exemple 1) et l'exemple comparatif sont reportées dans la figure 2.

On constate que le film de l'exemple 1 de l'invention, qui contient 13% (sous forme sèche) d'un polysiloxane oxyéthyléné (Silsoft 880) a présenté une cinétique de dissolution très nettement améliorée par rapport à l'exemple comparatif.

Exemple de composition aqueuse dans laquelle peut être dissous le film.

### Composition aqueuse (sérum)

| *Phase A* | |
|---|---|
| Gomme de xanthane | 0,2 % |
| PVM/MA Decadiene Crosspolymer | 0,2 % |
| Methyl Paraben | 0,2 % |
| Phenoxyéthanol | 0,35 % |
| Eau | qsp 100 % |

| *Phase B* | |
|---|---|
| Triéthanolamine | 0,2 % |
| Polyacrylamide / C13-C14 isoparaffine / laureth-7 (Sepigel 305) | 1 % |
| Diazolidinyl Urea | 0,3 % |
| Glycérine | 7 % |

Mode opératoire de préparation du sérum : On a chauffé la phase A à 75°C environ, sous agitation, puis on a versé la phase B préalablement préparée dans la phase A. Ensuite, on a arrêté le chauffage tout en maintenant l'agitation jusqu'au retour à température ambiante. Une légère agitation a été ensuite maintenue pendant 30 minutes.

La composition aqueuse (sérum) et un ou plusieurs films de l'exemple 1 peuvent être présentés sous forme d'un kit Lors de l'utilisation, la consommatrice mélange dans le creux de sa main avec les doigts, une ou plusieurs films de l'exemple 1 avec une dose comprise entre 100 et 500 mg de la composition aqueuse pendant une dizaine de secondes. Elle masse légèrement la surface à traiter de manière à favoriser l'étalement du produit ainsi obtenu, sur la peau.

Le produit final obtenu peut être appliqué sur le visage ou le corps, notamment sur le visage pour tous types de traitements cosmétiques selon les actifs ajoutés à la composition, par exemple comme composition anti-âge ou anti-vieillissement, comme composition matifiante, notamment pour les peaux grasses, pour la dépigmentation de la peau, comme composition amincissante, pour la photoprotection de la peau, pour correger les dyschromies, pour donner un effet bonne mine, comme auto bronzant, etc....

## Revendications

1. Film anhydre hydrosoluble comportant (i) au moins un polymère filmogène hydrosoluble ou hydrodispersible, (ii) au moins un agent épaississant polysaccharidique, (iii) au moins un polysiloxane oxyalkyléné hydrosoluble ou hydrodispersible, et (iv) au moins un plastifiant choisi parmi les polyols.

2. Film selon la revendication 1, **caractérisé en ce qu'**il a une épaisseur de 10 µm à 1000 µm et de préférence de 20 à 500 µm.

3. Film selon la revendication 1 ou 2, **caractérisé en ce qu'**il contient 0 à 15 % d'eau.

4. Film selon l'une quelconque des revendications précédentes, **caractérisé en ce que** le polysiloxane oxyalkyléné a une solubilité dans l'eau, mesurée à 25°C, au moins égale à 0,1 gramme/litre.

5. Film selon l'une quelconque des revendications précédentes, **caractérisé en ce que** le polysiloxane oxyalkyléné est choisi parmi les silicones hydrosolubles de formule (a) :
R²₃SiO(R²₂SiO)ₚ(R²PESiO)_{q}SIR²₃ (a)
dans laquelle
- les radicaux R², identiques ou différents, désignent un radical hydrocarboné monovalent choisi parmi les radicaux alkyles, aryles et aralkyles ayant au plus 10 atomes de carbone ;
- p varie de 0 à 150, de préférence de 0 à 100 ;
- q varie de 1 à 12, de préférence de 1 à 10 ;
- le groupe polyéther PE a la formule (b) suivante
-CₓH₂ₓ(OC₂H₄)_{y}(OC₃H₆)_{z}OR³ (b)
dans laquelle :
- x varie de 1 à 8 et de préférence varie de 2 à 4 ;
- y est supérieur à 0,
- z est supérieur ou égal à 0 ; les valeurs de y et z étant tels que le poids moléculaire total de la portion polyoxyalkylénée du groupe polyéther PE varie de 200 à 10.000 ;
- R³ désigne l'hydrogène, un groupe alkyle en C₁-C₈ ou un groupe acyle en C₂-C₈ .

6. Film selon la revendication précédente, **caractérisé en ce que**, dans la formule (a), les radicaux R² sont choisis parmi les groupes alkyles en C₁-C₄ et les groupes hexyle, phényle et benzyle.

7. Film selon la revendication 5 ou 6, **caractérisé en ce que**, dans la formule (a), les radicaux R³ sont choisis parmi les groupes alkyles en C₁-C₄.

8. Film selon l'une quelconque des revendications 5 à 7, **caractérisé en ce que** les silicones hydrosolubles sont de formule (a') :
MeSiO(MeSiO)ₚ(MePESiO)_{q}SIMe₃ (a')
dans laquelle :
- p varie de 0 à 150, de préférence de 0 à 100 ;
- q varie de 1 à 12, de préférence de 1 à 10 ;
- Me désigne le radical méthyle ;
- PE désigne :
-(CH₂)₃O(OC₂H₄)_{y}(OC₃H₆)_{z}OR³ (b')
dans laquelle :
- y est supérieur à 0,
- z est supérieur ou égal à 0 ; les valeurs de y et z étant tels que le poids moléculaire total de la portion polyoxyalkylénée du groupe polyéther PE varie de 200 à 10.000 ;
- R³ désigne hydrogène ou un groupe alkyle en C₁-C₄.

9. Film selon l'une quelconque des revendications 1 à 4, **caractérisé en ce que** le polysiloxane oxyalkyléné est choisi parmi silicones ramifiées de formule (c) suivante :
(MeSiO)_{q-2}[SiOMe2)_{p/q} OPE]_{q} (c)
dans laquelle :
- p varie de 0 à 150, de préférence de 0 à 100 ;
- q varie de 1 à 12, de préférence de 1 à 10 ;
- Me désigne le radical méthyle ;
- PE désigne le groupe de formule (d) suivante :
-(OC₂H₄)_{y}(OC₃H₆)_{z}R³ (d)
dans laquelle :
- y est supérieur à 0,
- z est supérieur ou égal à 0 ; les valeurs de y et z étant tels que le poids moléculaire total de la portion polyoxyalkylénée du groupe polyéther PE varie de 200 à 10.000 et plus préférentiellement de 350 à 4000 ;
- R³ désigne un groupe alkyle en C₁-C₄.

10. Film selon l'une quelconque des revendications précédentes, **caractérisé en ce que** la quantité de polysiloxane(s) oxyalkyléné(s) va de 0,5 à 30 % en poids, de préférence de 1 à 20 % en poids par rapport au poids total du film.

11. Film selon l'une quelconque des revendications précédentes, **caractérisé en ce que** le polymère filmogène a une solubilité dans l'eau, mesurée à 25°C au moins égale à 0,1 gramme/litre.

12. Film selon l'une quelconque des revendications précédentes, **caractérisé en ce que** le polymère filmogène est choisi parmi les polymères vinyliques, les dérivés cellulosiques, les amidons et leurs dérivés, les polymères d'origine naturelle, éventuellement modifiés, les polymères dérivant de la chitine ou du chitosane, les polymères protéiques, les copolymères acryliques de phosphoryle choline, les complexes anion-cation, et leurs mélanges.

13. Film selon l'une quelconque des revendications précédentes, **caractérisé en ce que** le polymère filmogène est choisi parmi les polymères vinyliques, les dérivés cellulosiques et leurs mélanges.

14. Film selon la revendication précédente, **caractérisé en ce que** le polymère filmogène est choisi parmi l'acétate de polyvinyle, l'hydroxypropylcellulose, l'hydroxypropylméthylcellulose et leurs mélanges.

15. Film selon l'une quelconque des revendications précédentes, **caractérisé en ce que** la quantité de polymère(s) filmogène(s) hydrosoluble(s) va de 10 à 95 % en poids et de préférence de 20 à 70 % en poids par rapport au poids total du film.

16. Film selon l'une quelconque des revendications précédentes, **caractérisé en ce que** l'agent épaississant polysaccharidique est choisi parmi la gomme arabique, la gomme de ghatti, la gomme de karaya, la gomme de caroube, la gomme de guar, la gomme de tamarin, la gomme de xanthane, la gellane les pectines, le tragacanth, l'agar, les alginates, le carrageenan, le furcelleran, le konjac, les dérivés de cellulose, et leurs mélanges.

17. Film selon l'une quelconque des revendications précédentes, **caractérisé en ce que** l'agent épaississant polysaccharidique est choisi parmi les carraghénanes.

18. Film selon l'une quelconque des revendications précédentes, **caractérisé en ce que** la quantité d'agent(s) épaississant(s) va de 0,5 à 40 % en poids, de préférence de 1 à 20 % en poids par rapport au poids total du film.

19. Film selon l'une quelconque des revendications précédentes, **caractérisé en ce que** la quantité de polyol(s) plastifiant(s) va de 1 à 40 % en poids et de préférence de 2 à 15 % en poids par rapport au poids total du film.

20. Produit cosmétique obtenu par mélange extemporané d'au moins un film anhydre hydrosoluble selon l'une quelconque des revendications précédentes, avec une quantité appropriée d'une composition aqueuse.

21. Produit selon la revendication précédente, **caractérisé en ce que** la composition aqueuse se présente sous forme de solution ou d'émulsion.

22. Produit selon la revendication 20 ou 21, **caractérisé en ce que** le ou les films fins et/ou la composition aqueuse contiennent un ou plusieurs actifs.

23. Kit de formulation d'un produit cosmétique comprenant (1) une composition aqueuse, et (2) au moins un film anhydre hydrosoluble selon l'une quelconque des revendications 1 à 19.

24. Kit de formulation d'un produit cosmétique, notamment de soin ou de maquillage, adaptée à la demande du consommateur, comprenant :
i) une composition aqueuse ;
ii) une pluralité de films anhydres hydrosolubles selon l'une quelconque des revendications 1 à 19, identiques ou différents, destinés à être mélangé)s à la composition aqueuse pour former ledit produit cosmétique, et
iii) éventuellement des instructions, notamment sur une notice explicative, pour formuler à façon ledit produit cosmétique en fonction du nombre de films anhydres hydrosolubles identiques ou différents à mélanger à la composition.
